# EUROPEAN PATENT APPLICATION

(11) **EP 4 400 606 A1**
(43) Date of publication of application: **17.07.2024**
(21) Application number: 22886398.1
(22) Date of filing: 12.07.2022
(51) Int. Cl.: C21B 5/00, C07C 1/12, C21B 7/00, F27D 17/00, F27D 19/00

(54) **METHOD FOR OPERATING METHANE GAS GENERATION DEVICE, METHOD FOR OPERATING BLAST FURNACE, METHOD FOR PRODUCING METHANE GAS, METHOD FOR PRODUCING HOT METAL, AND METHANE GAS GENERATION DEVICE**

(30) Priority: 29.10.2021 JP 2021178334
(71) Applicant: JFE Steel Corporation, Tokyo 100-0011 (JP)
(72) Inventor: TAKAHASHI, Koichi, Tokyo 100-0011 (JP); NOUCHI, Taihei, Tokyo 100-0011 (JP); KASHIHARA, Yusuke, Tokyo 100-0011 (JP); KAWASHIRI, Yuki, Tokyo 100-0011 (JP); MORITA, Yuya, Tokyo 100-0011 (JP)
(74) Representative: Haseltine Lake Kempner LLP
(86) International application number: PCT/JP2022/027483
(87) International publication number: WO 2023/074061

(57) **Abstract**

A method of operating a methane gas generation device and a method of operating a blast furnace that make it possible to control the deterioration rate of a catalyst by appropriately adjusting the operating conditions of the blast furnace and the methane gas generation device. A method of operating a methane gas generation device includes: a process of generating methane gas, using a methane gas synthesis reactor, from raw material gas including blast furnace gas discharged from a blast furnace; a process of obtaining methane concentration in the methane gas; and a process of adjusting methane gas generation conditions based on a comparison between the methane concentration and a predetermined methane concentration target value.

## Description

### TECHNICAL FIELD

The present disclosure relates to a method of operating a methane gas generation device, a method of operating a blast furnace, a method of producing methane gas, a method of producing hot metal, and a methane gas generation device.

### BACKGROUND

In recent years, there has been a strong demand to reduce carbon dioxide (CO₂) emissions against a backdrop of global environmental issues. Therefore, there is a demand for low reducing agent rate (low RAR) operations in the operation of blast furnaces in steelworks.

In a typical blast furnace, hot blast (air heated to about 1200 °C) is blown into the blast furnace as blast gas from a tuyere. This causes the oxygen in the hot blast to react with coke or pulverized coal that serves as the reducing agent, generating carbon monoxide (CO) gas and hydrogen (H₂) gas. The carbon monoxide gas and hydrogen gas reduce the iron ore charged in the blast furnace. Further, carbon dioxide is generated in this iron ore reduction reaction. The blast gas is gas that is blown into the blast furnace through the tuyere. The blast gas also plays a role in gasifying pulverized coal and coke in the blast furnace.

As a technology to reduce carbon dioxide emissions from such blast furnace operations, a method has been proposed to synthesize methane from carbon monoxide, carbon dioxide, and hydrogen as raw material gas included in by-product gas emitted from blast furnaces and the like, and reuse the methane as a blast furnace reducing agent.

For example, the following is described in Patent Literature (PTL) 1: "a blast furnace operating method comprising: Process (A) for separating and collecting CO₂ and/or CO from mixed gas containing CO₂ and/or CO; Process (B) for adding hydrogen to CO₂ and/or CO separated and collected in Process (A) and converting CO₂ and/or CO into CH₄; Process (C) for separating and removing H₂O from gas obtained after Process (B); and Process (D) for blowing the gas after Process (C) into the blast furnace".

Further, the following is described in PTL 2: "a method of operating a blast furnace, comprising: generating a regenerative methane gas from a by-product gas discharged from the blast furnace; and blowing a blast gas and a reducing agent into the blast furnace from a tuyere, wherein the blast gas is oxygen gas and the regenerative methane gas is used as at least part of the reducing agent".

### CITATION LIST

### Patent Literature

PTL 1: JP 2011-225969 A
PTL 2: WO 2021/106578 A1

### SUMMARY

### (Technical Problem)

Using the methods described in PTL 1 and 2, methane may be synthesized from CO/CO₂ gas and hydrogen gas in blast furnace gas and used as a reducing agent, and therefore these are effective methods for reducing CO₂ emissions. On the other hand, to synthesize methane from CO/CO₂ gas and hydrogen gas, use of a catalyst such as nickel to speed up the reaction is essential, but catalysts deteriorate when exposed to high temperatures, reducing reaction efficiency. In particular, the methane synthesis reaction from CO/CO₂ gas and hydrogen gas is a reaction that generates a large amount of heat, so the synthesis reaction is carried out while controlling the temperature using a powerful cooling mechanism such as a shell-and-tube system. However, complete and uniform temperature control of the synthesis reactor is very difficult, and localized hot spots occurring and resulting in catalyst deterioration is unavoidable. In addition, blast furnaces only have periodic repairs once every one to three months, and otherwise operate continuously, day and night, making it difficult to shut down a facility for catalyst replacement even when the catalyst deteriorates.

It would be helpful to provide a method of operating a methane gas generation device and a method of operating a blast furnace that make it possible to control the deterioration rate of a catalyst by appropriately adjusting the operating conditions of the blast furnace and the methane gas generation device.

### (Solution to Problem)

As a result of diligent research to solve the technical problems faced by conventional technology, the inventors have developed a new method of operating a methane gas generation device and a new method of operating a blast furnace as described below.
[1] A method of operating a methane gas generation device, comprising:
   a process of generating methane gas using a methane gas synthesis reactor from raw material gas including blast furnace gas discharged from a blast furnace;
   a process of obtaining methane concentration in the methane gas; and
   a process of adjusting methane gas generation conditions based on a comparison between the methane concentration and a predetermined methane concentration target value.
[2] The method of operating a methane gas generation device according to [1] above, wherein the methane concentration in the methane gas generated in the process of generating methane gas is adjusted based on the comparison.
[3] The method of operating a methane gas generation device according to [1] or [2] above, wherein a flow rate ratio of the blast furnace gas introduced into the methane gas synthesis reactor in the process of generating methane gas is adjusted based on the comparison.
[4] The method of operating a methane gas generation device according to any one of [1] to [3] above, wherein the raw material gas further includes hydrogen gas, and a flow rate ratio of the hydrogen gas introduced into the methane gas synthesis reactor in the process of generating methane gas is adjusted based on the comparison.
[5] The method of operating a methane gas generation device according to any one of [1] to [4] above, wherein a reaction ratio of the methane synthesis reaction in the process of generating methane gas is adjusted based on the comparison.
[6] The method of operating a methane gas generation device according to any one of [1] to [5] above, wherein the raw material gas further includes inert gas and concentration of the inert gas in the raw material gas is adjusted based on the comparison.
[7] The method of operating a methane gas generation device according to any one of [1] to [6] above, wherein an amount of ammonia added to the raw material gas is adjusted based on the comparison.
[8] A method of operating a blast furnace using the method of operating a methane gas generation device according to any one of [1] to [7] above, comprising:
   a process of charging blast furnace raw material into the blast furnace;
   a process of blowing blast gas and reducing agent into the blast furnace; and
   a process of discharging the blast furnace gas from the blast furnace, wherein
   the methane gas is used as at least a part of the reducing agent.
[9] The method of operating a blast furnace according to [8] above, wherein an operating condition of the blast furnace is adjusted based on the comparison.
[10] The method of operating a blast furnace according to [9] above, wherein inert gas concentration in the blast gas is adjusted based on the comparison.
[11] The method of operating a blast furnace according to [9] or [10] above, wherein inert gas is further blown into the blast furnace and a blowing amount of the inert gas is adjusted based on the comparison.
[12] The method of operating a blast furnace according to any one of [9] to [11] above, wherein ammonia is further blown into the blast furnace and a blowing amount of ammonia is adjusted based on the comparison.
[13] A method of producing methane gas using the method of operating a methane gas generation device according to any one of [1] to [7] above.
[14] A method of producing hot metal using the method of operating a blast furnace according to any one of [8] to [12] above.
[15] A methane gas generation device for generating methane gas from raw material gas including blast furnace gas, comprising:
   a methane gas synthesis reactor configured to generate methane gas from the raw material gas;
   a methane concentration meter configured to obtain methane concentration in the methane gas generated; and
   a control unit that adjusts methane gas generation conditions based on a comparison between the methane concentration and a predetermined methane concentration target value.

### (Advantageous Effect)

The present disclosure provides a method of operating a methane gas generation device and a method of operating a blast furnace that make it possible to control the deterioration rate of a catalyst by appropriately adjusting the operating conditions of the blast furnace and the methane gas generation device.

### BRIEF DESCRIPTION OF THE DRAWINGS

In the accompanying drawings:
FIG. 1 is a diagram schematically illustrating an example of a blast furnace and a blast furnace ancillary facility such as a methane gas generation device used in a method of operating a methane gas generation device according to an embodiment of the present disclosure;
FIG. 2 is a diagram schematically illustrating an example of a blast furnace and a blast furnace ancillary facility such as a methane gas generation device used in a method of operating a methane gas generation device according to an embodiment of the present disclosure;
FIG. 3 is a diagram schematically illustrating an example of a blast furnace and a blast furnace ancillary facility such as a methane gas generation device used in a method of operating a methane gas generation device according to an embodiment of the present disclosure;
FIG. 4 is a diagram schematically illustrating an example of a blast furnace and a blast furnace ancillary facility such as a methane gas generation device used in a method of operating a methane gas generation device according to an embodiment of the present disclosure;
FIG. 5 is a diagram schematically illustrating an example of a blast furnace and a blast furnace ancillary facility such as a methane gas generation device used in a method of operating a methane gas generation device according to an embodiment of the present disclosure;
FIG. 6 is a diagram schematically illustrating an example of a blast furnace and a blast furnace ancillary facility such as a methane gas generation device used in a method of operating a methane gas generation device according to an embodiment of the present disclosure; and
FIG. 7 is a diagram schematically illustrating an example of a blast furnace and a blast furnace ancillary facility such as a methane gas generation device used in a method of operating a methane gas generation device according to an embodiment of the present disclosure.

### DETAILED DESCRIPTION

The following describes embodiments of the present disclosure. However, the present disclosure is not limited to the following embodiments.

A method of operating a methane gas generation device includes: a process of generating methane gas from raw material gas including blast furnace gas discharged from a blast furnace; a process of obtaining methane concentration in the methane gas; and a process of adjusting methane gas generation conditions based on a comparison between the methane concentration and a predetermined methane concentration target value. Accordingly, in the process of generating methane gas, the methane concentration in the methane gas synthesis reactor may be adjusted, and a deterioration rate of a catalyst due to reaction heat of the methane synthesis reaction may be controlled. As a result, catalyst life required by an operating schedule or the like of the blast furnace may be attained.

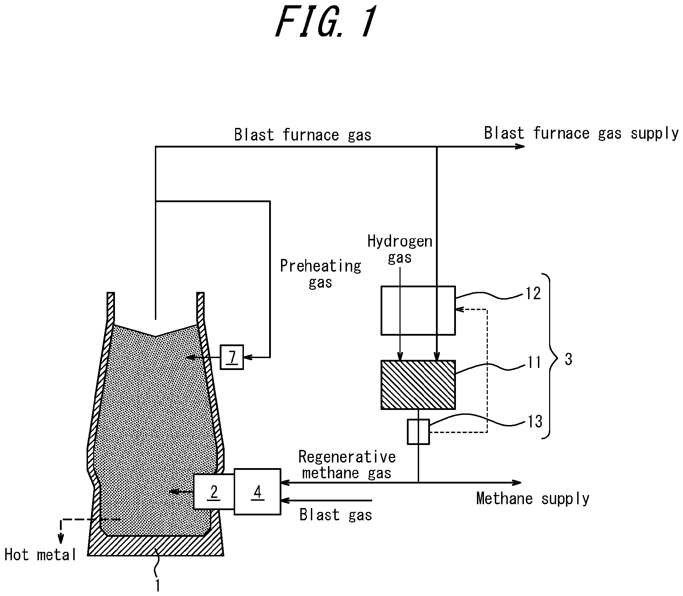

FIG. 1 is a schematic diagram illustrating an example of a blast furnace and a blast furnace ancillary facility such as a methane gas generation device to which a method of operating a blast furnace and the method of operating a methane gas generation device may be applied. The method of operating a blast furnace and the method of operating a methane gas generation device according to an embodiment of the present disclosure are described as an example applied to the blast furnace illustrated in FIG. 1. In the drawings, reference sign 1 indicates a blast furnace, 2 indicates a tuyere, 3 indicates a methane gas generation device, 4 indicates a gas blowing device, and 7 indicates a burner. Here, the term blast furnace includes shaft-type reduction furnaces and the like.

In the method of operating a blast furnace according to an embodiment of the present disclosure, raw material such as sintered ore, lump ore, pellets (hereinafter also referred to as "ore material"), coke, and the like are charged (not illustrated) into the furnace from the throat of the blast furnace 1. Hereinafter, the ore material and coke are also referred to as "blast furnace raw material". Further, blast gas and reducing agent are blown into the blast furnace 1 from the tuyere 2 installed at a bottom portion of the blast furnace. The reducing agent blown into the blast furnace 1 from the tuyere 2 may also be referred to as blown-in reducing agent to distinguish from coke. Carbon monoxide and hydrogen gas generated by the reaction between the blast gas and the reducing agent reduce and melt the ore material charged into the blast furnace 1 to produce hot metal. Carbon dioxide is generated in this reduction reaction of the ore material. The carbon dioxide, along with carbon monoxide and hydrogen that did not react with the ore material, is discharged from the throat of the blast furnace 1 as by-product gas.

A top portion of a blast furnace is normally under a high-pressure condition of about 2.5 atm. When the by-product gas discharged from the throat of the blast furnace 1 (hereinafter also referred to as "blast furnace gas") returns to normal pressure, water vapor is condensed by expansion and cooling, generating condensate. A blast furnace ancillary facility may include a dehydration device to dehydrate such condensate.

Next, raw material gas including at least a portion of the blast furnace gas is introduced into the methane gas generation device 3. In the methane gas generation device 3, carbon monoxide and carbon dioxide included in the blast furnace gas react with hydrogen to generate methane (CH₄) gas. Here, methane gas obtained by reacting blast furnace gas may also be referred to as regenerative methane gas. The hydrogen used to generate the regenerative methane gas may be supplied from within the steelworks or from outside. When hydrogen gas is produced in the steelworks, use of a process that produces as little carbon dioxide as possible is preferable. As such a production method, electrolysis of water is an example. Then, by cooling the regenerative methane gas to room temperature, water vapor in the regenerative methane gas is condensed to produce condensate. A blast furnace ancillary facility may include a dehydration device to dehydrate such condensate.

The regenerative methane gas is then introduced into the gas blowing device 4. The gas blowing device 4 is connected to the methane gas generation device 3. The gas blowing device 4 includes a methane gas supply that introduces into the tuyere 2 the regenerative methane gas that serves as blown-in reducing agent, and a blast gas supply that introduces blast gas into the tuyere 2 of the blast furnace 1.

When another blown-in reducing agent is used, the other blown-in reducing agent may also be introduced into the methane gas supply. When pulverized coal or waste plastic is used as the other blown-in reducing agent, a separate reducing agent supply for distributing pulverized coal or waste plastic may be included that is distinct from the methane gas supply.

All of the methane gas blown into the blast furnace 1 from the tuyere 2 (hereinafter also referred to as blown-in methane gas) need not be regenerative methane gas, and methane gas supplied from another line (hereinafter also referred to as external methane gas) may be used, in accordance with steelworks operations. In such a case, an external methane gas supply line may be connected to the methane gas supply of the gas blowing device 4, or to another reducing agent supply as described above. As an external methane gas, methane gas derived from fossil fuels is an example.

Regenerative methane gas may be generated from a portion of the blast furnace gas, and surplus blast furnace gas may be supplied into the steelworks. Further, when there is a surplus of regenerative methane gas, the surplus may be supplied into the steelworks. Blowing amounts of oxygen gas and reducing agent and other operating conditions are not particularly limited and may be appropriately determined according to the capacity of the blast furnace and other factors.

When the oxygen concentration in the blast gas increases, the temperature of the blast furnace raw material in the upper portion of the blast furnace 1 may be insufficiently raised. In such a case, as illustrated in FIG. 1, preheating gas blowing is preferably performed, in which a portion of the blast furnace gas is partially combusted by the burner 7 to reach about 800 °C to 1000 °C and then blown into the blast furnace shaft.

### [Methane gas generation device]

According to an example, the methane gas generation device 3 includes a methane gas synthesis reactor 11 and a methane concentration meter 13. The methane gas generation device 3 may further include a flow rate ratio regulator 12, as illustrated in FIG. 1. The methane gas synthesis reactor 11 generates regenerative methane gas from raw material gas including blast furnace gas. The flow rate ratio regulator 12 is an example of a control unit that adjusts conditions for methane gas generation, and adjusts the flow rate ratio of the raw material gas such as blast furnace gas introduced into methane gas synthesis reactor 11. The methane concentration meter 13 obtains the methane concentration in the methane gas generated. Further, the methane gas generation device 3, according to an example, includes a blast furnace gas intake and a hydrogen gas intake. When ammonia is added and hydrogen is generated from the ammonia, the methane gas generation device 3 does not need to include the hydrogen gas intake.

In the methane gas synthesis reactor 11, carbon monoxide and carbon dioxide in the blast furnace gas react with hydrogen in the hydrogen gas to generate regenerative methane gas. In the methane gas synthesis reactor 11, a catalyst such as Ni is used to increase the reaction rate, but such catalysts have a problem with deterioration phenomena such as sintering at high temperatures, resulting in a decrease in the reaction rate. The methane synthesis reaction involves a large amount of heat generation, and therefore appropriately cooling the methane gas synthesis reactor 11 in methane synthesis is extremely important. The methane gas synthesis reactor 11 is cooled by a shell-and-tube heat exchanger, for example. However, even when a heat exchanger with high heat exchange efficiency is used, completely avoiding non-uniform catalyst filling, foreign matter entering the coolant channels, or the like is difficult, and such factors may not allow for completely uniform cooling of the entire area inside the methane gas synthesis reactor 11. Therefore, a certain amount of hot spot occurrence is unavoidable, and the hot spots cause catalyst deterioration, requiring a certain frequency of shutdowns and repair work to replace the catalyst. On the other hand, a blast furnace is a process that operates continuously without stopping day and night, and large-scale repair work that stops production is only performed once every one to three months. Therefore, in a process where methane synthesis and the blast furnace are linked, shutting down the methane gas synthesis reactor 11 when the methane synthesis catalyst has deteriorated is inefficient. Ideally, life of the catalyst is extended and operation continues until the timing of large-scale repair work on the blast furnace, and the catalyst in the methane gas synthesis reactor 11 is replaced coinciding with the timing of the blast furnace shutdown.

Therefore, in the method of operating the methane gas generation device according to the present embodiment, the methane concentration in the generated methane gas is obtained, and the methane gas generation conditions are adjusted based on a comparison between the methane concentration and a predetermined methane concentration target value. This allows the methane concentration in the methane gas synthesis reactor 11 to be adjusted in the process of generating methane gas, and the deterioration rate of a catalyst due to the reaction heat of the methane synthesis reaction to be controlled. As a result, catalyst life required by an operating schedule or the like of the blast furnace may be attained. As methane gas generation conditions to be adjusted, examples include the flow rate ratio of blast furnace gas introduced into the methane gas generation process, the flow rate ratio of hydrogen gas introduced into the methane gas generation process, the reaction ratio of the methane synthesis reaction, the concentration of inert gas in the raw material gas, the amount of ammonia added to the raw material gas, and the like. These generation conditions may be adjusted singly or in combination.

According to an example, the methane concentration in the methane gas synthesis reactor 11 is adjusted to match the predetermined methane concentration based on the comparison between the methane concentration in the methane gas and the predetermined methane concentration target value. By adjusting the methane concentration in the methane gas synthesis reactor 11 to the predetermined concentration, the raw material gas and methane gas in the methane gas synthesis reactor 11 are appropriately diluted, and the methane concentration in the synthesis gas and the temperature rise of the catalyst in the methane gas synthesis reactor 11 may be appropriately controlled according to required conditions. For example, when productivity of the blast furnace is desired, the methane concentration in the methane gas synthesis reactor 11 may be increased so that a high concentration of methane may be blown into the blast furnace. On the other hand, when the catalyst deteriorates faster than expected and is unlikely to maintain catalytic function until the blast furnace is repaired, the methane concentration in the methane gas synthesis reactor 11 is lowered and the concentration of unreacted gas or inert gas is increased instead to moderate the temperature rise in the methane gas synthesis reactor 11. This allows the life of the catalyst to be extended until an appropriate timing for operational requirements, such as when blast furnace repairs are scheduled.

The methane concentration in the methane gas synthesis reactor 11 is preferably adjusted so that the difference between the predetermined methane concentration and the methane concentration in the methane gas synthesis reactor 11 is in ±10 % of the predetermined methane concentration, based on the comparison between the methane concentration in the methane gas and the predetermined methane concentration target value. More preferably, the methane concentration is adjusted to be in ±5 %.

Specific methods for adjusting the methane concentration in methane gas are not particularly limited. According to an example, the flow rate ratio regulator 12 adjusts the flow rate ratio of blast furnace gas introduced into the methane gas generation device 3 in the process of generating methane gas to adjust the methane concentration in the methane gas, based on the comparison between the methane concentration and the predetermined methane concentration target value. To reduce the methane concentration in the methane gas, according to an example, a flow rate ratio of blast furnace gas and hydrogen gas that allows 100 % methane synthesis by mixing blast furnace gas and hydrogen gas is defined as a "theoretical ratio", and the methane gas generation device 3 is operated under conditions where the blast furnace gas or hydrogen gas is in excess of the theoretical ratio. At such time, excess CO/CO₂ gas or hydrogen gas remains in the regenerative methane gas generated, which reduces the methane concentration in the methane gas synthesis reactor 11.

Based on the comparison between the methane concentration to the predetermined methane concentration target value, the flow rate ratio of hydrogen gas introduced into the methane gas generation device 3 in the process of generating methane gas may be adjusted. The methane concentration in the methane gas may be adjusted by adjusting the flow rate ratio of hydrogen gas introduced into the methane gas generation device 3 in the process of generating methane gas. According to an example, the flow rate ratio regulator 12 is used to adjust the flow rate ratio of the blast furnace gas introduced into the methane gas synthesis reactor 11. To reduce the methane concentration in the methane gas, according to an example, a flow rate ratio of blast furnace gas and hydrogen gas that allows 100 % methane synthesis by mixing blast furnace gas and hydrogen gas is defined as a "theoretical ratio", and the methane gas generation device 3 is operated under conditions where the hydrogen gas is in excess of the theoretical ratio. At such time, excess CO/CO₂ gas or hydrogen gas remains in the regenerative methane gas generated, which reduces the methane concentration in the methane gas synthesis reactor 11.

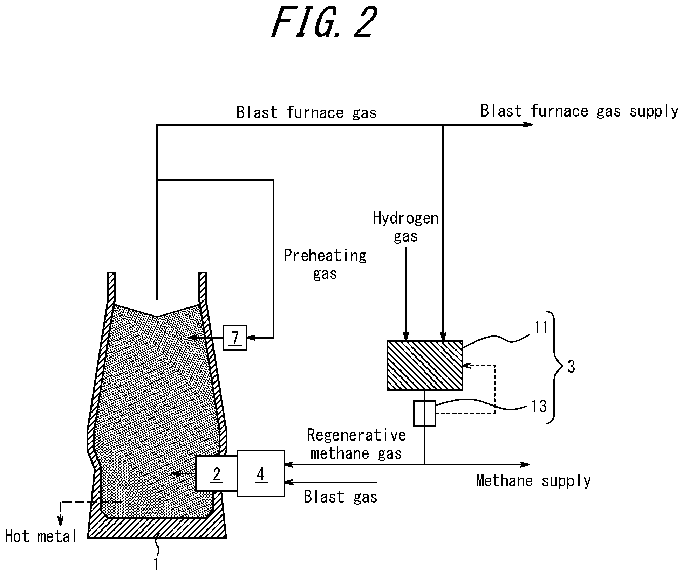

The methane concentration in the methane gas synthesis reactor 11 may be adjusted by adjusting the reaction ratio of the methane synthesis reaction based on the comparison between the methane concentration and the predetermined methane concentration target value. According to an example, as illustrated in FIG. 2, the reaction ratio of the methane synthesis reaction is adjusted based on the comparison between the methane concentration obtained by the methane concentration meter 13 and the predetermined methane concentration target value. For example, by operating the methane synthesis reaction in such a way that the reaction ratio is reduced and some of the blast furnace gas and hydrogen gas remain unreacted, unreacted CO, CO₂, and hydrogen gas will remain in the regenerative methane gas, resulting in a lower methane concentration in the methane gas synthesis reactor 11. Methods to reduce the reaction ratio of the methane synthesis reaction are not particularly limited. For example, the number of parallel reactors of the methane gas synthesis reactor 11 may be reduced to decrease the residence time of the raw material gas in the methane gas synthesis reactor 11, thereby reducing the reaction ratio of the methane synthesis reaction. When the methane gas synthesis reactor 11 includes 10 reactors, these 10 reactors may be connected in parallel, and each reactor may be individually configured to introduce and stop the raw material gas with a valve. The number of parallel reactors of the methane gas synthesis reactor 11 may be adjusted by opening and closing the valves.

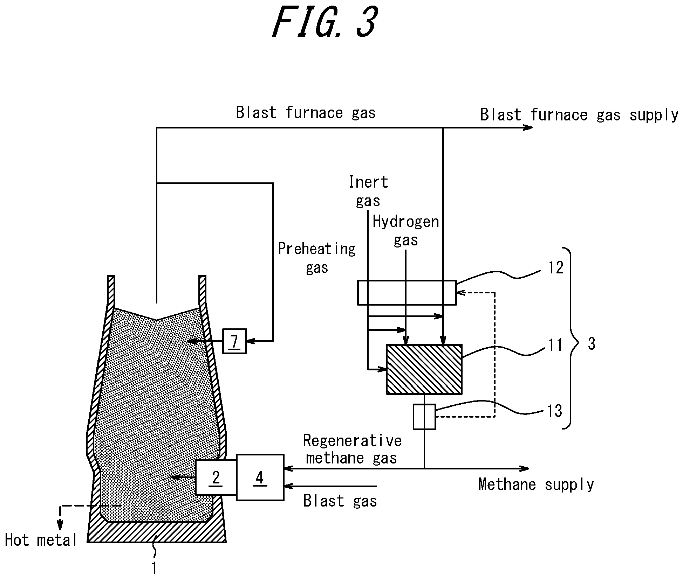

Further, the methane concentration in the methane gas synthesis reactor 11 may be adjusted by adding inert gas to the raw material gas and adjusting the concentration of the inert gas in the raw material gas. According to an example, as illustrated in FIG. 3, the concentration of the inert gas in the raw material gas is adjusted by adding the inert gas to at least one of the blast furnace gas or hydrogen gas based on the comparison between the methane concentration obtained by the methane concentration meter 13 and the predetermined methane concentration target value. As the inert gas to be mixed with the raw material gas, at least one of nitrogen, helium, or argon may be used. Such inert gases may be added to the raw material gas singly or in combination. A method of adding inert gas to the raw material gas is not particularly limited. For example, inert gas may be added to the blast furnace gas or hydrogen gas to adjust the inert gas concentration in the raw material gas. Specifically, the inert gas may be added to the blast furnace gas to adjust the inert gas concentration, or the inert gas may be mixed with blast furnace gas discharged from another blast furnace. Similarly, the inert gas may be added to hydrogen gas to adjust the inert gas concentration in the hydrogen gas. In such a case, the inert gas such as nitrogen may be mixed with hydrogen gas produced by electrolysis or natural gas reforming, or the inert gas concentration in the hydrogen gas may be adjusted using off-gas including nitrogen and hydrogen that is generated when CO₂ is separated from the blast furnace gas.

An amount of ammonia added to the raw material gas may be adjusted based on the comparison between the methane concentration and the predetermined methane concentration target value. This allows the methane concentration in the methane gas synthesis reactor 11 to be adjusted. Ammonia is decomposed into nitrogen and hydrogen in the methane gas synthesis reactor 11.

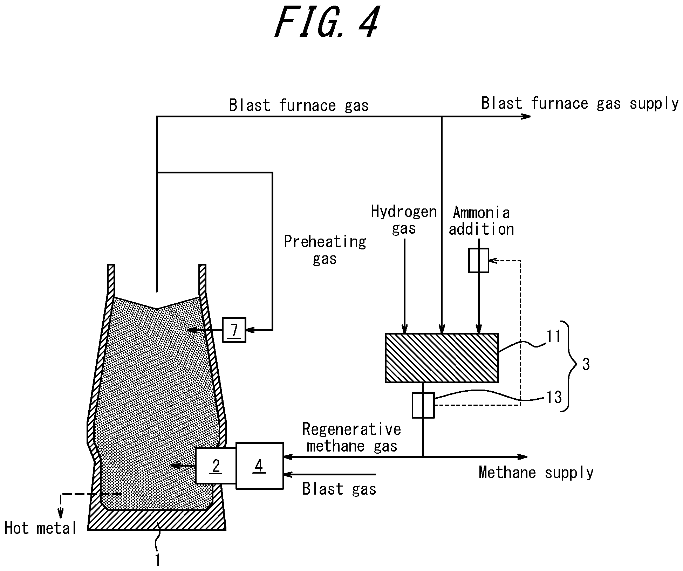

Further, the methane gas synthesis reactor 11 itself may include a separate inlet for inert gas, and the inert gas, blast furnace gas from another blast furnace, or ammonia may flow directly into the methane gas synthesis reactor 11 so that these gases mix in the methane gas synthesis reactor 11. According to an example, as illustrated in FIG. 4, the amount of ammonia added to the raw material gas is adjusted by adjusting the amount of ammonia that flows into the methane gas synthesis reactor 11 based on the comparison between the methane concentration obtained by the methane concentration meter 13 and the predetermined methane concentration target value.

When methane gas generation conditions are adjusted by using excess blast furnace gas or hydrogen gas and by reducing the reaction ratio, unreacted gases such as CO, CO₂, and H₂ included in the blast furnace gas or hydrogen gas that serves as dilution gas are included in the regenerative methane gas. These unreacted gases may be effectively utilized in the blast furnace without waste. The synthesized regenerative methane gas is blown into the blast furnace 1 through the tuyere 2 as a reducing agent, while CO and hydrogen gas function as reducing gas in the blast furnace as is. Further, CO₂ gas reacts with coke in a high-temperature space in front of the tuyere and is converted to CO gas, which functions as a reducing gas in the furnace as in the case of CO gas, although heat loss occurs during the gasification reaction.

The following is a description of a method of adjusting the methane concentration in the methane gas by adjusting operating conditions of the blast furnace 1. The operating conditions of the blast furnace 1 may be adjusted based on the comparison between the methane concentration in the methane gas and the predetermined methane concentration target value. As mentioned above, the blast furnace gas discharged from the blast furnace 1 is used as raw material gas to generate methane gas. The composition and the like of the discharged blast furnace gas, and methane gas generation conditions, may be adjusted by adjusting the operating conditions of the blast furnace 1, based on the comparison between the methane concentration in the methane gas and the predetermined methane concentration target value.

The operating conditions of the blast furnace 1 to be adjusted based on the comparison between the methane concentration in the methane gas and the predetermined methane concentration target value are not particularly limited. As operating conditions of the blast furnace 1 to be adjusted, examples include the inert gas concentration in the blast gas, the blowing amount of inert gas, the blowing amount of ammonia, and the like.

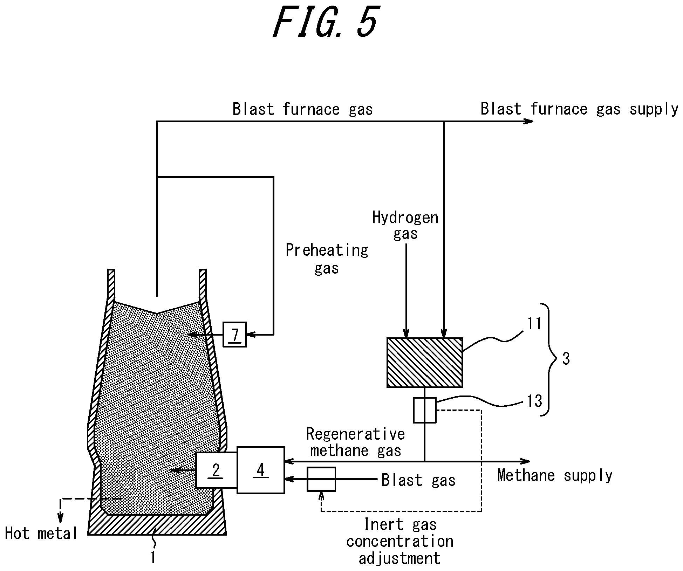

According to an example, the inert gas concentration in the blast gas to be blown into the blast furnace 1 is adjusted based on the comparison between the methane concentration in the methane gas and the predetermined methane concentration target value. By adjusting the inert gas concentration in the blast gas blown in from the tuyere 2 of the blast furnace 1, oxygen concentration in the blast gas is adjusted and the inert gas concentration in the blast furnace gas may be adjusted. According to an example, as illustrated in FIG. 5, the inert gas concentration in the blast gas blown in from the tuyere 2 of the blast furnace 1 is adjusted based on the comparison between the methane concentration obtained by the methane concentration meter 13 and the predetermined methane concentration target value. For example, by decreasing the inert gas concentration in the blast gas blown in from the tuyere 2 of the blast furnace 1, the oxygen concentration in the blast gas is increased and the inert gas concentration in the blast furnace gas may be decreased. Thus, the inert gas concentration in the blast furnace gas is adjusted by adjusting the inert gas concentration in the blast gas, which accordingly adjusts the inert gas concentration introduced into the methane gas synthesis reactor 11.

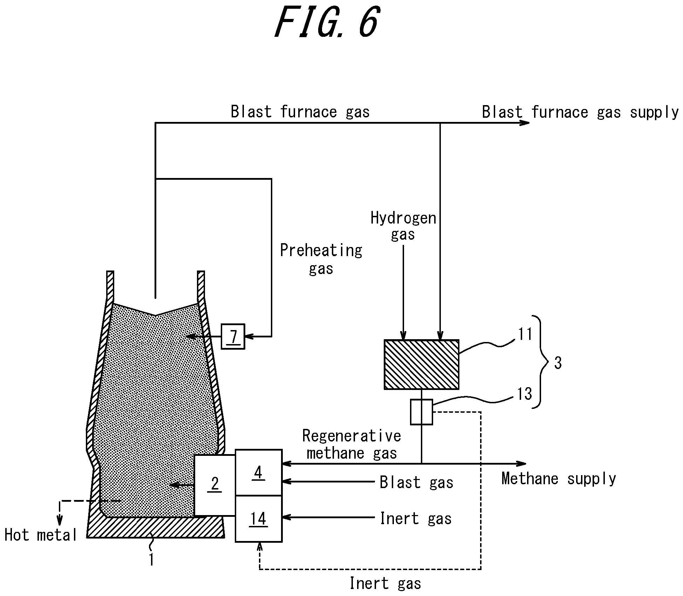

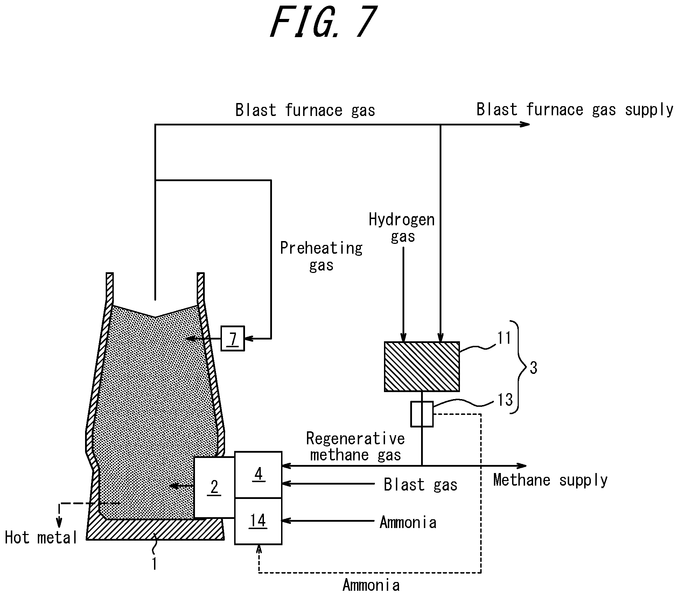

Further, a separate blowing device from the gas blowing device 4 may be further included, and the amount of gas blown in from the blowing device may be adjusted based on the comparison between the methane concentration in the methane gas with the predetermined methane concentration target value. As gas blown from the blowing device, examples include inert gas, ammonia, and mixtures of these gases. According to an example, as illustrated in FIG. 6, the amount of the inert gas blown in from a blowing device 14 is adjusted based on the comparison between the methane concentration obtained by the methane concentration meter 13 and the predetermined methane concentration target value. According to an example, as illustrated in FIG. 7, the amount of ammonia blown in from the blowing device 14 is adjusted based on the comparison between the methane concentration obtained by the methane concentration meter 13 and the predetermined methane concentration target value. The inert gas concentration in the blast furnace gas may be adjusted by adjusting the amount of gas blown in from the blowing device 14 based on the comparison between the methane concentration in the methane gas and the predetermined methane concentration target value. In the case of an operation that blows in powder such as pulverized coal from the tuyere 2, an inert gas such as nitrogen or air may be used as a carrier gas for powder blowing, and the amount of inert gas blown in from the tuyere 2 may be adjusted by increasing or decreasing the amount of carrier gas.

The methane concentration target value used for the comparison with the methane concentration is predetermined. The methane concentration target value suitable for regulating catalyst life varies from facility to facility and cannot be determined in general. Therefore, predetermining an appropriate methane concentration target value for each facility is preferable. The methane concentration target value may be changed over time. For example, an ideal change over time pattern of methane concentration in synthesized methane gas may be created based on actual operation data or the like, and the pattern may be used as the methane concentration target value. For example, the methane concentration in the regenerative methane gas generated may be monitored over time, and change over time of the methane concentration may be compared to the change over time pattern of the methane concentration target value, and the methane gas generation conditions may be adjusted based on the comparison. For example, when the methane gas concentration decreases at a faster rate than the ideal change over time pattern of a methane concentration target value, the catalyst is considered to be deteriorating and operation may be controlled to lower the methane gas concentration and approach the ideal change over time pattern of methane concentration.

The method for obtaining the methane concentration in the methane gas generated is not particularly limited. For example, as illustrated in FIG. 1, the methane concentration in the methane gas at the outlet of the methane gas synthesis reactor 11 may be obtained using the methane concentration meter 13. The type of methane concentration meter 13 is also not particularly limited, and any known meter may be applied, such as a meter using a gas chromatograph, for example. Further, the methane concentration in the methane gas does not have to be obtained by direct analysis. When the reaction ratio in the methane gas synthesis reactor 11 may be estimated from other measurements, such as temperature and pressure drop of the methane gas synthesis reactor 11, the methane concentration in the methane gas may be obtained by a mass balance calculation without directly analyzing the methane concentration in the methane gas generated.

The regenerative methane gas synthesized in the methane gas synthesis reactor may be only enough to be blown into the blast furnace 1, and surplus blast furnace gas may be used as a heat source for other facilities and the like. As illustrated in FIG. 1, more regenerative methane gas may be generated than the amount to be blown into the blast furnace 1, and surplus regenerative methane gas may be used in other facilities or sold externally. A theoretical maximum temperature in the methane gas synthesis reactor 11 will not change when the flow rate ratio of the raw material gas, blast furnace gas and hydrogen gas, is constant, and therefore surplus regenerative methane gas may be produced by increasing the gas flow rate while keeping the flow rate ratio of blast furnace gas and hydrogen gas constant.

As described above, in the method of operating a methane gas generation device and the method of operating a blast furnace according to the present embodiment, the methane concentration in the methane gas generated using the raw material gas including the blast furnace gas is obtained, and the methane gas generation conditions are adjusted based on the comparison between the methane concentration and the predetermined methane concentration target value. Accordingly an effect is obtained that the methane concentration in the methane gas synthesis reactor 11 is adjusted to control catalyst deterioration caused by the heat of the methane synthesis reaction and catalyst life required by the operation schedule of the blast furnace 1 may be attained. Further, the dilution gas introduced into the methane gas synthesis reactor 11 is blast furnace gas, hydrogen gas, inert gas including CO, CO₂, H₂, N₂, and the like. Such gas functions as a tuyere-outlet temperature control gas and as a reducing gas when blown into the blast furnace with the synthesized methane, and therefore an effect is obtained that such gas may be used without waste after serving as dilution gas in the methane gas synthesis reactor 11.

### EXAMPLES

FIG. 1 to FIG. 7 schematically illustrate the blast furnace and blast furnace ancillary facility used for Examples of the present disclosure. According to the present Examples, the blast furnace is an oxygen blast furnace into which pure oxygen is blown, except for Examples 20 to 22, in which the inert gas concentration in the blast gas is changed. Regenerative methane gas was produced in a methane gas synthesis reactor from a portion of the blast furnace gas and externally supplied hydrogen gas, and the methane gas was blown in as a reducing agent via the tuyere of the blast furnace. The basic flow is that a portion of the blast furnace gas generated from the blast furnace flows back into the methane gas synthesis reactor as raw material for methane synthesis.

Examples 1 to 7 are examples of blast furnace operations using the blast furnace and blast furnace ancillary facility illustrated in FIG. 1. In Example 1, the reaction ratio of the methane gas synthesis reactor is 100 %, and the flow rate ratio between blast furnace gas and hydrogen gas is an example of adjusting the methane gas production conditions so that the methane concentration in the regenerative methane gas produced is 100 %. Under these conditions, the only inert gas in the blast furnace gas is derived from the trace nitrogen in the coke, and the inert gas concentration in the blast furnace gas is almost zero. Hydrogen gas is also pure hydrogen with zero inert gas concentration. Accordingly, the inert gas concentration in the regenerative methane gas is almost zero. At this time, the maximum temperature that can occur in the methane gas synthesis reactor may be considered as the post-reaction temperature under adiabatic conditions with zero cooling of the methane gas synthesis reactor, and therefore hot spot temperature may be evaluated by heat mass balance calculation before and after the reaction. The theoretical maximum temperature in the methane gas synthesis reactor under the conditions of Example 1 was calculated to be 1412 °C.

When the operating conditions of the methane gas synthesis reactor are adjustable to lower the theoretical maximum temperature, the hot spot temperature in the methane gas synthesis reactor may be lowered and the catalyst life may be extended. Therefore, in the following Examples, whether the theoretical maximum temperature in the methane gas synthesis reactor could be controlled by adjusting the methane concentration to a predetermined target value was checked.

### [Examples 2 to 6]

Examples 2 to 6 illustrate examples of operations in which the methane concentration in the regenerative methane gas is reduced by adjusting the flow rate ratio of blast furnace gas to lower the temperature in the methane gas synthesis reactor. For each of the Examples, Table 1 lists the operating conditions of the blast furnace and methane gas synthesis reactor and the theoretical maximum temperature of the reactor calculated by heat mass balance calculation.

The following is a description of a theoretical maximum temperature calculation method by heat mass balance calculation. From the principle of thermodynamics, molar flow rate of each atom such as C, H, and O is the same before and after the methane gas synthesis reaction, and enthalpy flow rate before and after the reaction is also the same. Therefore, first, the molar flow rate of each component in the regenerative methane gas, that is, methane, hydrogen, and the like, may be calculated from each atomic molar flow rate of C, H, O, and the like of the raw material gas entering the methane gas synthesis reactor, and reaction ratio. Then, by using the principle that the enthalpy flow rate is the same before and after the reaction for the calculated composition and flow rate of the regenerative methane gas, the temperature of the regenerative methane gas, or theoretical maximum temperature, may be calculated assuming no reactor cooling at all. This calculation requires thermodynamic data such as standard enthalpy of formation and specific heat of each component in raw material gas and regenerative methane gas such as methane and hydrogen. For example, for thermodynamic data, the NIST Chemistry WebBook is available from the US National Institute of Standards and Technology (NIST).

### [Table 1]

**Table 1**

| | | | | Example 1 | Example 2 | Example 3 | Example 4 | Example 5 | Example 6 | Example 7 |
|---|---|---|---|---|---|---|---|---|---|---|
| Blast furnace specifications | Coke rate | | kg/t | 358 | 364 | 370 | 382 | 398 | 446 | 502 |
| | Tuyere blowing | Regenerative methane blowing amount | Nm³/t | 200 | 265 | 330 | 461 | 199 | 198 | 195 |
| | | Other type of blown-in material | - | - | - | - | - | - | - | - |
| | | Other blown-in material blowing amount | Nm³/t | - | - | - | - | - | - | - |
| | | Tuyere-outlet temperature | °C | 2100 | 2100 | 2100 | 2100 | 2100 | 2100 | 2100 |
| | Blast | Flow rate | Nm³/t | 331 | 319 | 308 | 285 | 328 | 326 | 324 |
| | | Temperature | °C | 25 | 25 | 25 | 25 | 25 | 25 | 25 |
| | | Oxygen concentration | vol% | 100 | 100.0 | 100.0 | 100.0 | 100.0 | 100.0 | 100.0 |
| | | Inert gas concentration | vol% | 0 | 0.0 | 0.0 | 0.0 | 0.0 | 0.0 | 0.0 |
| | Blast furnace gas | Amount generated | Nm³/t | 881 | 873 | 864 | 847 | 901 | 928 | 965 |
| Methane synthesis | Blast furnace gas | Amount used | Nm³/t | 256 | 217 | 177 | 88 | 239 | 222 | 206 |
| | | Inert gas concentration | vol% | 0 | 0.4 | 0.4 | 0.4 | 0.5 | 0.5 | 0.5 |
| | Hydrogen gas | Amount used | Nm³/t | 642 | 624 | 609 | 586 | 513 | 359 | 176 |
| | | Inert gas concentration | vol% | 0 | 0.0 | 0.0 | 0.0 | 0.0 | 0.0 | 0.0 |
| | Ammonia | Amount used | Nm³/t | 0 | 0 | 0 | 0 | 0 | 0 | 0 |
| | | Inert gas concentration | vol% | 0 | 0.0 | 0.0 | 0.0 | 0.0 | 0.0 | 0.0 |
| | Regenerative methane gas | Amount generated | Nm³/t | 200 | 265 | 330 | 461 | 199 | 198 | 195 |
| | | Methane concentration | vol% | 99.4 | 61.9 | 39.2 | 13.1 | 78.6 | 54.7 | 26.9 |
| | | Inert gas concentration | vol% | 1 | 0.4 | 0.2 | 0.1 | 0.6 | 0.6 | 0.6 |
| | | CO concentration | vol% | 0 | 0.0 | 0.0 | 0.0 | 9.8 | 20.6 | 32.0 |
| | | CO₂ concentration | vol% | 0 | 0.0 | 0.0 | 0.0 | 11.0 | 24.2 | 40.5 |
| | | Hydrogen concentration | vol% | 0 | 37.7 | 60.6 | 86.8 | 0.0 | 0.0 | 0.0 |
| | | Methane concentration target value | vol% | 100 | 60 | 40 | 15 | 80 | 55 | 25 |
| | Reactor | Reaction ratio | % | 100 | 100 | 100 | 100 | 100 | 100 | 100 |
| | | By-product water generation | kg/t | 242 | 200 | 158 | 74 | 192 | 134 | 66 |
| | | Theoretical maximum temperature | °C | 1412 | 1275 | 1108 | 636 | 1320 | 1162 | 830 |
| | | Temperature drop | °C | - | 137 | 304 | 776 | 92 | 250 | 582 |

Examples 2 to 4 are examples of operations in which the flow rate ratio of blast furnace gas is adjusted to an excess of hydrogen gas. In Examples 2 to 4, the ratio of hydrogen gas to blast furnace gas was increased in this order. In Examples 2 to 4, the reaction ratio of the methane gas synthesis reactor was 100 % in all cases. While the methane concentration in the regenerative methane gas was almost 100 vol% in Example 1, the methane concentration in the regenerative methane gas in Examples 2 to 4 was reduced to 61.9 vol%, 39.2 vol%, and 13.1 vol%, respectively. The theoretical maximum temperature decreased as the methane concentration decreased, and the theoretical maximum temperatures in Examples 2 to 4 decreased from 1412 °C in Example 1 to 1275 °C, 1108 °C, and 636 °C, respectively. These results confirm that the theoretical maximum temperature of the methane gas synthesis reactor is controllable by increasing the flow rate ratio of hydrogen gas, thereby controlling the deterioration rate of the catalyst.

Examples 5 to 7 are examples of operations in which the flow rate ratio of blast furnace gas is adjusted to an excess of blast furnace gas. In Examples 5 to 7, the ratio of blast furnace gas to hydrogen gas was increased in this order. In Examples 5 to 7, the reaction ratio of the methane gas synthesis reactor was 100 % in all cases. While the methane concentration in the regenerative methane gas was almost 100 % in Example 1, the methane concentration in the regenerative methane gas in Examples 5 to 7 was reduced to 78.6 vol%, 54.7 vol%, and 26.9 vol%, respectively. The theoretical maximum temperature decreased as the methane concentration decreased, and the theoretical maximum temperatures in Examples 5 to 7 decreased from 1412 °C in Example 1 to 1320 °C, 1162 °C, and 830 °C, respectively. These results confirm that the theoretical maximum temperature of the methane gas synthesis reactor is controllable by adjusting the flow rate ratio of the blast furnace gas, thereby controlling the deterioration rate of the catalyst.

### [Examples 8 to 10]

Examples 8 to 10 are examples of blast furnace operations using the blast furnace and the blast furnace ancillary facility illustrated in FIG. 2. In these Examples, the number of parallel reactors of the methane gas synthesis reactor was reduced to decrease the methane gas synthesis reactor reaction ratio by decreasing the residence time of the raw material gas in the methane gas synthesis reactor. The operating conditions of the blast furnace and methane gas synthesis reactor at this time and the theoretical maximum temperature of the reactor calculated by the heat mass balance calculation are listed in Table 2.

### [Table 2]

**Table 2**

| | | | | Example 8 | Example 9 | Example 10 |
|---|---|---|---|---|---|---|
| Blast furnace specifications | Coke rate | | kg/t | 379 | 396 | 431 |
| | Tuyere blowing | Regenerative methane blowing amount | Nm³/t | 240 | 273 | 341 |
| | | Other type of blown-in material | - | - | - | - |
| | | Other blown-in material blowing amount | Nm³/t | - | - | - |
| | | Tuyere-outlet temperature | °C | 2100 | 2100 | 2100 |
| | Blast | Flow rate | Nm³/t | 323 | 316 | 302 |
| | | Temperature | °C | 25 | 25 | 25 |
| | | Oxygen concentration | vol% | 100.0 | 100.0 | 100.0 |
| | | Inert gas concentration | vol% | 0.0 | 0.0 | 0.0 |
| | Blast furnace gas | Amount generated | Nm³/t | 883 | 885 | 890 |
| Methane synthesis | Blast furnace gas | Amount used | Nm³/t | 225 | 201 | 151 |
| | | Inert gas concentration | vol% | 0.5 | 0.5 | 0.5 |
| | Hydrogen gas | Amount used | Nm³/t | 576 | 522 | 407 |
| | | Inert gas concentration | vol% | 0.0 | 0.0 | 0.0 |
| | Ammonia | Amount used | Nm³/t | 0 | 0 | 0 |
| | | Inert gas concentration | vol% | 0.0 | 0.0 | 0.0 |
| | Regenerative methane gas | Amount generated | Nm³/t | 240 | 273 | 341 |
| | | Methane concentration | vol% | 66.3 | 46.8 | 18.1 |
| | | Inert gas concentration | vol% | 0.4 | 0.3 | 0.2 |
| | | CO concentration | vol% | 3.5 | 5.6 | 8.6 |
| | | CO₂ concentration | vol% | 3.8 | 6.1 | 9.5 |
| | | Hydrogen concentration | vol% | 25.9 | 41.2 | 63.7 |
| | | Methane concentration target value | vol% | 65 | 45 | 20 |
| | Reactor | Reaction ratio | % | 90 | 80 | 50 |
| | | By-product water generation | kg/t | 195 | 156 | 76 |
| | | Theoretical maximum temperature | °C | 1286 | 1157 | 744 |
| | | Temperature drop | °C | 126 | 255 | 668 |

In Examples 8 to 10, the reaction ratio of the methane synthesis reaction was reduced in this order. The methane concentration in the regenerative methane gas decreased as the reaction ratio decreased, and the methane concentrations in the regenerative methane gas in Examples 8 to 10 were 66.3 vol%, 46.8 vol%, and 18.1 vol%, respectively. The theoretical maximum temperature decreased as the methane concentration decreased, and the theoretical maximum temperatures in Examples 8 to 10 decreased from 1412 °C in Example 1 to 1286 °C, 1157 °C, and 744 °C, respectively. These results confirm that the theoretical maximum temperature of the methane gas synthesis reactor is controllable by adjusting the reaction ratio of the reactor, thereby controlling the deterioration rate of the catalyst.

### [Examples 11 to 16]

Examples 11 to 16 are examples of blast furnace operations using the blast furnace and the blast furnace ancillary facility illustrated in FIG. 3. These Examples illustrate examples of operations in which the inert gas is mixed with the blast furnace gas and hydrogen gas to increase the inert gas concentration of the blast furnace gas and hydrogen gas. The operating conditions of the blast furnace and methane gas synthesis reactor at this time and the theoretical maximum temperature of the reactor calculated by the heat mass balance calculation are listed in Table 3.

### [Table 3]

**Table 3**

| | | | | Example 11 | Example 12 | Example 13 | Example 14 | Example 15 | Example 16 |
|---|---|---|---|---|---|---|---|---|---|
| Blast furnace specifications | Coke rate | | kg/t | 377 | 411 | 531 | 377 | 455 | 531 |
| | Tuyere blowing | Regenerative methane blowing amount | Nm³/t | 248 | 334 | 641 | 248 | 448 | 641 |
| | | Other type of blown-in material | - | - | - | - | - | - | - |
| | | Other blown-in material blowing amount | Nm³/t | - | - | - | - | - | - |
| | | Tuyere-outlet temperature | °C | 2100 | 2100 | 2100 | 2100 | 2100 | 2100 |
| | Blast | Flow rate | Nm³/t | 332 | 333 | 339 | 332 | 335 | 339 |
| | | Temperature | °C | 25 | 25 | 25 | 25 | 25 | 25 |
| | | Oxygen concentration | vol% | 100.0 | 100.0 | 100.0 | 100.0 | 100.0 | 100.0 |
| | | Inert gas concentration | vol% | 0.0 | 0.0 | 0.0 | 0.0 | 0.0 | 0.0 |
| | Blast furnace gas | Amount generated | Nm³/t | 941 | 1049 | 1439 | 941 | 1192 | 1439 |
| Methane synthesis | Blast furnace gas | Amount used | Nm³/t | 293 | 363 | 642 | 243 | 160 | 42 |
| | | Inert gas concentration | vol% | 23.4 | 52.1 | 96.3 | 7.6 | 29.5 | 43.2 |
| | Hydrogen gas | Amount used | Nm³/t | 587 | 484 | 81 | 637 | 340 | 81 |
| | | Inert gas concentration | vol% | 0.0 | 0.0 | 0.0 | 7.9 | 46.9 | 88.2 |
| | Ammonia | Amount used | Nm³/t | 0 | 0 | 0 | 0 | 0 | 0 |
| | | Inert gas concentration | vol% | 0.0 | 0.0 | 0.0 | 0.0 | 0.0 | 0.0 |
| | Regenerative methane gas | Amount generated | Nm³/t | 248 | 334 | 641 | 248 | 447 | 641 |
| | | Methane concentration | vol% | 72.4 | 43.4 | 3.6 | 72.4 | 22.3 | 3.6 |
| | | Inert gas concentration | vol% | 27.6 | 56.6 | 96.4 | 27.6 | 77.7 | 96.4 |
| | | CO concentration | vol% | 0.0 | 0.0 | 0.0 | 0.0 | 0.0 | 0.0 |
| | | CO₂ concentration | vol% | 0.0 | 0.0 | 0.0 | 0.0 | 0.0 | 0.0 |
| | | Hydrogen concentration | vol% | 0.0 | 0.0 | 0.0 | 0.0 | 0.0 | 0.0 |
| | | Methane concentration target value | vol% | 70 | 45 | 5 | 70 | 20 | 5 |
| | Reactor | Reaction ratio | % | 100 | 100 | 100 | 100 | 100 | 100 |
| | | By-product water generation | kg/t | 220 | 179 | 29 | 220 | 124 | 29 |
| | | Theoretical maximum temperature | °C | 1316 | 1128 | 230 | 1316 | 842 | 230 |
| | | Temperature drop | °C | 96 | 284 | 1182 | 96 | 570 | 1182 |

Examples 11 to 13 illustrate examples of operations in which nitrogen is added as the inert gas to the blast furnace gas to increase the inert gas concentration in the raw material gas. In Examples 11 to 13, the concentration of nitrogen relative to the blast furnace gas was increased in this order. In Examples 11 to 13, the inert gas concentration in the blast furnace gas was 23.4 vol%, 52.1 vol%, and 96.3 vol%, respectively (blast furnace gas concentration was 76.6 vol%, 47.9 vol%, and 3.7 vol%, respectively). The reaction ratio of the methane gas synthesis reactor was 100 % in all cases. In Examples 11 to 13, the methane concentration in the regenerative methane gas was reduced to 72.4 vol%, 43.4 vol%, and 3.6 vol%, respectively. The theoretical maximum temperature decreased as the methane concentration decreased, and the theoretical maximum temperatures in Examples 11 to 13 decreased from 1412 °C in Example 1 to 1316 °C, 1128 °C, and 230 °C, respectively. These results confirm that the theoretical maximum temperature of the methane gas synthesis reactor is controllable by adjusting the inert gas concentration in the raw material gas, thereby controlling the deterioration rate of the catalyst.

Examples 14 to 16 illustrate examples of operations in which nitrogen is added to hydrogen gas as the inert gas to increase the inert gas concentration in the raw material gas. In Examples 14 to 16, the concentration of nitrogen relative to hydrogen gas was increased in this order. In Examples 14 to 16, the inert gas concentration in hydrogen gas was 7.6 vol%, 29.5 vol%, and 43.2 vol%, respectively (hydrogen gas concentration was 92.4 vol%, 70.5 vol%, and 56.8 vol%, respectively). The reaction ratio of the methane gas synthesis reactor was 100 % in all cases. In Examples 14 to 16, the methane concentration in the regenerative methane gas was reduced to 72.4 vol%, 22.3 vol%, and 3.6 vol%, respectively. The theoretical maximum temperature decreased as the methane concentration decreased, and the theoretical maximum temperatures in Examples 14 to 16 decreased from 1412 °C in Example 1 to 1316 °C, 842 °C, and 230 °C, respectively. These results confirm that the theoretical maximum temperature of the methane gas synthesis reactor is controllable by adjusting the inert gas concentration in the raw material gas, thereby controlling the deterioration rate of the catalyst.

### [Examples 17 to 19]

Examples 17 to 19 are examples of blast furnace operations using the blast furnace and the blast furnace ancillary facility illustrated in FIG. 4. Examples 17 to 19 illustrate examples of operations in which ammonia gas is added to the blast furnace gas and hydrogen gas as the raw material gas for the methane gas synthesis reactor. For each of the Examples, Table 4 lists the operating conditions of the blast furnace and methane gas synthesis reactor and the theoretical maximum temperature of the reactor calculated by heat mass balance calculation.

### [Table 4]

**Table 4**

| | | | | Example 17 | Example 18 | Example 19 |
|---|---|---|---|---|---|---|
| Blast furnace specifications | Coke rate | | kg/t | 377 | 414 | 415 |
| | Tuyere blowing | Regenerative methane blowing amount | Nm³/t | 248 | 341 | 365 |
| | | Other type of blown-in material | - | - | - | - |
| | | Other blown-in material blowing amount | Nm³/t | - | - | - |
| | | Tuyere-outlet temperature | °C | 2100 | 2100 | 2100 |
| | Blast | Flow rate | Nm³/t | 332 | 333 | 328 |
| | | Temperature | °C | 25 | 25 | 25 |
| | | Oxygen concentration | vol% | 100.0 | 100.0 | 100.0 |
| | | Inert gas concentration | vol% | 0.0 | 0.0 | 0.0 |
| | Blast furnace gas | Amount generated | Nm³/t | 941 | 1057 | 1049 |
| Methane synthesis | Blast furnace gas | Amount used | Nm³/t | 243 | 210 | 192 |
| | | Inert gas concentration | vol% | 7.6 | 19.1 | 18.7 |
| | Hydrogen gas | Amount used | Nm³/t | 437 | 0 | 0 |
| | | Inert gas concentration | vol% | 0.0 | 0.0 | 0.0 |
| | Ammonia | Amount used | Nm³/t | 100 | 317 | 314 |
| | | Inert gas concentration | vol% | 0.0 | 0.0 | 0.0 |
| | Regenerative methane gas | Amount generated | Nm³/t | 248 | 341 | 365 |
| | | Methane concentration | vol% | 72.4 | 41.7 | 35.3 |
| | | Inert gas concentration | vol% | 27.6 | 58.3 | 52.9 |
| | | CO concentration | vol% | 0.0 | 0.0 | 0.0 |
| | | CO₂ concentration | vol% | 0.0 | 0.0 | 0.0 |
| | | Hydrogen concentration | vol% | 0.0 | 0.0 | 11.7 |
| | | Methane concentration target value | vol% | 70 | 40 | 35 |
| | Reactor | Reaction ratio | % | 100 | 100 | 100 |
| | | By-product water generation | kg/t | 220 | 176 | 159 |
| | | Theoretical maximum temperature | °C | 1171 | 574 | 482 |
| | | Temperature drop | °C | 241 | 838 | 930 |

Example 17 is an example of operation using the blast furnace gas, hydrogen gas, and ammonia as the raw material gas for the methane gas synthesis reactor. In this example of operation, the blast furnace gas and hydrogen gas flow rates are adjusted to account for the hydrogen gas content generated by decomposition of ammonia in the reactor, so that no CO/CO₂ gas or hydrogen gas remains in the regenerative methane gas after the reaction. Example 18 is an example of an operation in which hydrogen produced by the decomposition reaction of ammonia gas replaces all the raw material hydrogen gas for the methane synthesis reaction. Only blast furnace gas and ammonia are fed into the methane gas synthesis reactor as raw material gas, and there is no inflow of hydrogen gas. Example 19 is an example of an operation in which excess ammonia is fed into the methane gas synthesis reactor. In this case, there was surplus hydrogen even when the amount of hydrogen gas fed was reduced to zero, resulting in residual hydrogen in the regenerative methane gas after synthesis. Under all conditions of Examples 17 to 19, nitrogen produced by the decomposition reaction of ammonia remained in the regenerative methane gas, and this nitrogen caused the methane concentration to decrease. In Examples 17 to 19, the methane concentration in the regenerative methane gas was 72.4 vol%, 41.7 vol%, and 35.3 vol%, respectively. The theoretical maximum temperature decreased as the methane concentration decreased, and the theoretical maximum temperatures in Examples 17 to 19 decreased from 1412 °C in Example 1 to 1171 °C, 574 °C, and 482 °C, respectively. These results confirm that the theoretical maximum temperature of the methane gas synthesis reactor is controllable by adjusting the amount of ammonia gas added to the raw material gas, thereby controlling the deterioration rate of the catalyst.

### [Examples 20 to 22]

Examples 20 to 22 are examples of blast furnace operations using the blast furnace and the blast furnace ancillary facility illustrated in FIG. 5. In these Examples, the inert gas concentration in the blast gas is increased. For each of the Examples, Table 5 lists the operating conditions of the blast furnace and methane gas synthesis reactor and the theoretical maximum temperature of the reactor calculated by heat mass balance calculation.

### [Table 5]

**Table 5**

| | | | | Example 20 | Example 21 | Example 22 |
|---|---|---|---|---|---|---|
| Blast furnace specifications | Coke rate | | kg/t | 389 | 468 | 463 |
| | Tuyere blowing | Regenerative methane blowing amount | Nm³/t | 194 | 134 | 110 |
| | | Other type of blown-in material | - | - | - | - |
| | | Other blown-in material blowing amount | Nm³/t | - | - | - |
| | | Tuyere-outlet temperature | °C | 2100 | 2100 | 2100 |
| | Blast | Flow rate | Nm³/t | 416 | 675 | 1234 |
| | | Temperature | °C | 25 | 25 | 1200 |
| | | Oxygen concentration | vol% | 80.0 | 50.0 | 21.0 |
| | | Inert gas concentration | vol% | 20.0 | 50.0 | 79.0 |
| | Blast furnace gas | Amount generated | Nm³/t | 979 | 1226 | 1806 |
| Methane synthesis | Blast furnace gas | Amount used | Nm³/t | 233 | 144 | 114 |
| | | Inert gas concentration | vol% | 11.6 | 31.6 | 57.8 |
| | Hydrogen gas | Amount used | Nm³/t | 551 | 303 | 149 |
| | | Inert gas concentration | vol% | 0.0 | 0.0 | 0.0 |
| | Ammonia | Amount used | Nm³/t | 0 | 0 | 0 |
| | | Inert gas concentration | vol% | 0.0 | 0.0 | 0.0 |
| | Regenerative methane gas | Amount generated | Nm³/t | 194 | 134 | 110 |
| | | Methane concentration | vol% | 86.1 | 66.0 | 40.0 |
| | | Inert gas concentration | vol% | 13.9 | 34.0 | 60.0 |
| | | CO concentration | vol% | 0.0 | 0.0 | 0.0 |
| | | CO₂ concentration | vol% | 0.0 | 0.0 | 0.0 |
| | | Hydrogen concentration | vol% | 0.0 | 0.0 | 0.0 |
| | | Methane concentration target value | vol% | 85 | 65 | 40 |
| | Reactor | Reaction ratio | % | 100 | 100 | 100 |
| | | By-product water generation | kg/t | 205 | 110 | 52 |
| | | Theoretical maximum temperature | °C | 1367 | 1272 | 1115 |
| | | Temperature drop | °C | 45 | 140 | 297 |

In Examples 20 to 22, the oxygen concentration in the blast gas was reduced in this order. That is, in Examples 20 to 22, the nitrogen concentration in the blast gas was increased in this order. In Example 1, the inert gas concentration in the blast furnace gas was almost zero, but as the nitrogen concentration in the blast gas is increased in Examples 20 to 22, the nitrogen concentration in the blast furnace gas increased to 11.6 vol%, 31.6 vol%, and 57.8 vol%, respectively. As a result, the methane concentration in the regenerative methane gas decreased as the nitrogen concentration in the blast gas increased, and the methane concentrations in the regenerative methane gas in Examples 20 to 22 were 86.1 vol%, 66.0 vol%, and 40.0 vol%, respectively. The theoretical maximum temperatures in Examples 20 to 22 decreased from 1412 °C in Example 1 to 1367 °C, 1272 °C, and 1115 °C, respectively. These results confirm that the theoretical maximum temperature of the methane gas synthesis reactor is controllable by adjusting the inert gas concentration in the blast gas of the blast furnace, thereby controlling the deterioration rate of the catalyst.

### [Examples 23 and 24]

Examples 23 and 24 are examples of blast furnace operations using the blast furnace and the blast furnace ancillary facility illustrated in FIG. 6. In these Examples, nitrogen gas is blown as an inert gas from the blowing lance of the tuyere to increase the amount of inert gas blown into the tuyere. For each of the Examples, Table 6 lists the operating conditions of the blast furnace and methane gas synthesis reactor and the theoretical maximum temperature of the reactor calculated by heat mass balance calculation.

### [Table 6]

**Table 6**

| | | | | Example 23 | Example 24 |
|---|---|---|---|---|---|
| Blast furnace specifications | Coke rate | | kg/t | 389 | 468 |
| | Tuyere blowing | Regenerative methane blowing amount | Nm³/t | 194 | 134 |
| | | Other type of blown-in material | - | Nitrogen | Nitrogen |
| | | Other blown-in material blowing amount | Nm³/t | 83 | 337 |
| | | Tuyere-outlet temperature | °C | 2100 | 2100 |
| | Blast | Flow rate | Nm³/t | 333 | 337 |
| | | Temperature | °C | 25 | 25 |
| | | Oxygen concentration | vol% | 100.0 | 100.0 |
| | | Inert gas concentration | vol% | 0.0 | 0.0 |
| | Blast furnace gas | Amount generated | Nm³/t | 979 | 1226 |
| Methane synthesis | Blast furnace gas | Amount used | Nm³/t | 233 | 144 |
| | | Inert gas concentration | vol% | 11.6 | 31.6 |
| | Hydrogen gas | Amount used | Nm³/t | 551 | 303 |
| | | Inert gas concentration | vol% | 0.0 | 0.0 |
| | Ammonia | Amount used | Nm³/t | 0 | 0 |
| | | Inert gas concentration | vol% | 0.0 | 0.0 |
| | Regenerative methane gas | Amount generated | Nm³/t | 194 | 134 |
| | | Methane concentration | vol% | 86.1 | 66.0 |
| | | Inert gas concentration | vol% | 13.9 | 34.0 |
| | | CO concentration | vol% | 0.0 | 0.0 |
| | | CO₂ concentration | vol% | 0.0 | 0.0 |
| | | Hydrogen concentration | vol% | 0.0 | 0.0 |
| | | Methane concentration target value | vol% | 85 | 65 |
| | Reactor | Reaction ratio | % | 100 | 100 |
| | | By-product water generation | kg/t | 205 | 110 |
| | | Theoretical maximum temperature | °C | 1367 | 1272 |
| | | Temperature drop | °C | 45 | 140 |

In Examples 23 and 24, the amount of nitrogen gas blown into the blast furnace tuyere was increased in this order. In Example 1, the inert gas concentration in the blast furnace gas was almost zero, but as the amount of nitrogen gas blown in increased, the nitrogen concentration in the blast furnace gas in Examples 23 and 24 increased to 11.6 vol% and 31.6 vol%, respectively. As a result, the methane concentration in the regenerative methane gas decreased as the nitrogen concentration in the blast gas increased, and in Examples 23 and 24, the methane concentration in the regenerative methane gas was 86.1 vol% and 66.0 vol%, respectively. The theoretical maximum temperatures in Examples 23 and 24 decreased from 1412 °C in Example 1 to 1367 °C and 1272 °C, respectively. These results confirm that the theoretical maximum temperature of the methane gas synthesis reactor is controllable by adjusting the amount of inert gas blown into the tuyere of the blast furnace, thereby controlling the deterioration rate of the catalyst.

### [Examples 25 and 26]

Examples 25 and 26 are examples of blast furnace operations using the blast furnace and the blast furnace ancillary facility illustrated in FIG. 7. In these Examples, ammonia is blown in from the tuyere. For each of the Examples, Table 7 lists the operating conditions of the blast furnace and methane gas synthesis reactor and the theoretical maximum temperature of the reactor calculated by heat mass balance calculation.

### [Table 7]

**Table 7**

| | | | | Example 25 | Example 26 |
|---|---|---|---|---|---|
| Blast furnace specifications | Coke rate | | kg/t | 421 | 444 |
| | Tuyere blowing | Regenerative methane blowing amount | Nm³/t | 106 | 64 |
| | | Other type of blown-in material | - | Ammonia | Ammonia |
| | | Other blown-in material blowing amount | Nm³/t | 50 | 70 |
| | | Tuyere-outlet temperature | °C | 2100 | 2100 |
| | Blast | Flow rate | Nm³/t | 327 | 325 |
| | | Temperature | °C | 25 | 25 |
| | | Oxygen concentration | vol% | 100.0 | 100.0 |
| | | Inert gas concentration | vol% | 0.0 | 0.0 |
| | Blast furnace gas | Amount generated | Nm³/t | 947 | 968 |
| Methane synthesis | Blast furnace gas | Amount used | Nm³/t | 132 | 79 |
| | | Inert gas concentration | vol% | 8.3 | 10.6 |
| | Hydrogen gas | Amount used | Nm³/t | 308 | 180 |
| | | Inert gas concentration | vol% | 0.0 | 0.0 |
| | Ammonia | Amount used | Nm³/t | 0 | 0 |
| | | Inert gas concentration | vol% | 0.0 | 0.0 |
| | Regenerative methane gas | Amount generated | Nm³/t | 106 | 64 |
| | | Methane concentration | vol% | 89.6 | 86.9 |
| | | Inert gas concentration | vol% | 10.4 | 13.1 |
| | | CO concentration | vol% | 0.0 | 0.0 |
| | | CO₂ concentration | vol% | 0.0 | 0.0 |
| | | Hydrogen concentration | vol% | 0.0 | 0.0 |
| | | Methane concentration target value | vol% | 90 | 85 |
| | Reactor | Reaction ratio | % | 100 | 100 |
| | | By-product water generation | kg/t | 116 | 68 |
| | | Theoretical maximum temperature | °C | 1381 | 1372 |
| | | Temperature drop | °C | 31 | 40 |

In Examples 25 and 26, the amount of ammonia blown into the blast furnace tuyere was increased in this order. In Example 1, the inert gas concentration in the blast furnace gas was almost zero, but as the amount of ammonia blown in increased, the nitrogen concentration in the blast furnace gas in Examples 25 and 26 increased to 8.3 vol% and 10.6 vol%, respectively. As a result, the methane concentration in the regenerative methane gas decreased as the nitrogen concentration in the blast gas increased, and in Examples 25 and 26, the methane concentration in the regenerative methane gas was 89.6 vol% and 86.9 vol%, respectively. The theoretical maximum temperatures in Examples 25 and 26 decreased from 1412 °C in Example 1 to 1381 °C and 1372 °C, respectively. These results confirm that the theoretical maximum temperature of the methane gas synthesis reactor is controllable by adjusting the amount of ammonia blown into the tuyere of the blast furnace, thereby controlling catalyst life.

### REFERENCE SIGNS LIST

- 1: blast furnace
- 2: tuyere
- 3: methane gas generation device
- 4: gas blowing device
- 7: burner
- 11: methane gas synthesis reactor
- 12: flow rate ratio regulator
- 13: methane concentration meter
- 14: blowing device

## Claims

1. A method of operating a methane gas generation device, comprising:
a process of generating methane gas using a methane gas synthesis reactor from raw material gas including blast furnace gas discharged from a blast furnace;
a process of obtaining methane concentration in the methane gas; and
a process of adjusting methane gas generation conditions based on a comparison between the methane concentration and a predetermined methane concentration target value.

2. The method of operating a methane gas generation device according to claim 1, wherein the methane concentration in the methane gas generated in the process of generating methane gas is adjusted based on the comparison.

3. The method of operating a methane gas generation device according to claim 1 or 2, wherein a flow rate ratio of the blast furnace gas introduced into the methane gas synthesis reactor in the process of generating methane gas is adjusted based on the comparison.

4. The method of operating a methane gas generation device according to any one of claims 1 to 3, wherein the raw material gas further includes hydrogen gas, and a flow rate ratio of the hydrogen gas introduced into the methane gas synthesis reactor in the process of generating methane gas is adjusted based on the comparison.

5. The method of operating a methane gas generation device according to any one of claims 1 to 4, wherein a reaction ratio of the methane synthesis reaction in the process of generating methane gas is adjusted based on the comparison.

6. The method of operating a methane gas generation device according to any one of claims 1 to 5, wherein the raw material gas further includes inert gas and concentration of the inert gas in the raw material gas is adjusted based on the comparison.

7. The method of operating a methane gas generation device according to any one of claims 1 to 6, wherein an amount of ammonia added to the raw material gas is adjusted based on the comparison.

8. A method of operating a blast furnace using the method of operating a methane gas generation device according to any one of claims 1 to 7, comprising:
a process of charging blast furnace raw material into the blast furnace;
a process of blowing blast gas and reducing agent into the blast furnace; and
a process of discharging the blast furnace gas from the blast furnace, wherein
the methane gas is used as at least a part of the reducing agent.

9. The method of operating a blast furnace according to claim 8, wherein an operating condition of the blast furnace is adjusted based on the comparison.

10. The method of operating a blast furnace according to claim 9, wherein inert gas concentration in the blast gas is adjusted based on the comparison.

11. The method of operating a blast furnace according to claim 9 or 10, wherein inert gas is further blown into the blast furnace and a blowing amount of the inert gas is adjusted based on the comparison.

12. The method of operating a blast furnace according to any one of claims 9 to 11, wherein ammonia is further blown into the blast furnace and a blowing amount of ammonia is adjusted based on the comparison.

13. A method of producing methane gas using the method of operating a methane gas generation device according to any one of claims 1 to 7.

14. A method of producing hot metal using the method of operating a blast furnace according to any one of claims 8 to 12.

15. A methane gas generation device for generating methane gas from raw material gas including blast furnace gas, comprising:
a methane gas synthesis reactor configured to generate methane gas from the raw material gas;
a methane concentration meter configured to obtain methane concentration in the methane gas generated; and
a control unit that adjusts methane gas generation conditions based on a comparison between the methane concentration and a predetermined methane concentration target value.
